# EUROPÄISCHE PATENTSCHRIFT

(11) **EP 1 239 781 B1**
(45) Veröffentlichungstag und Bekanntmachung des Hinweises auf die Patenterteilung: **14.06.2006**
(21) Anmeldenummer: 00990814.6
(22) Anmeldetag: 22.12.2000
(51) Int. Cl.: A61B 17/115

(54) **VORRICHTUNG ZUR FUNKTIONSGERECHTEN HALTERUNG EINER GEGENDRUCKPLATTE FÜR EIN SCHAFTFÖRMIGES CHIRURGISCHES KLAMMERNAHTINSTRUMENT**
DEVICE FOR FUNCTIONAL MOUNTING OF A COUNTER-PRESSURE PLATE FOR A SHAFT-LIKE SURGICAL MECHANICAL SUTURING DEVICE
DISPOSITIF POUR FIXER DE MANIERE FONCTIONNELLE UNE PLAQUE DE CONTRE-PRESSION DESTINEE A UN INSTRUMENT CHIRURGICAL EN FORME DE TIGE, SERVANT A POSER DES AGRAFES

(30) Priorität: 23.12.1999 DE 19962581
(43) Veröffentlichungstag der Anmeldung: 18.09.2002
(73) Patentinhaber: Von Fuchs, Alexander, 5020 Salzburg (AT)
(72) Erfinder: Von Fuchs, Alexander, 5020 Salzburg (AT)
(74) Vertreter: Gallo, Wolfgang
(86) Internationale Anmeldenummer: PCT/EP2000/013172
(87) Internationale Veröffentlichungsnummer: WO 2001/047424

(56) Entgegenhaltungen:
- DE-A- 3 301 713
- US-A- 4 047 654
- US-A- 5 344 059
- US-A- 5 458 579

## Beschreibung

Die Erfindung betrifft eine Vorrichtung zur funktionsgerechten Halterung einer Gegendruckplatte für ein schaftförmiges chirurgisches Klammernahtinstrument, insbesondere für eine laparoskopische VBG (Vertikale Band-Gastroplastik) nach Mason, bei der ein Loch in die Magenvorderwand und Magenhinterwand gestanzt werden muß, welches mit Hilfe eines Circularstaplers durchgeführt wird.

Bei derartigen laparoskopischen Operationen wird ein mit einem Dorn bewehrter Stapler entweder von vom nach hinten oder umgekehrt durch die Magenvorder- und -hinterwand gestoßen und dann mit der Gegendruckplatte konnektiert. Über dieses Loch wird dann der Magenfundus abgetrennt und der Auslaß mit einem Band umwickelt, damit dieser nicht aufweiten kann. Wegen der großen Schwierigkeit, die Gegendruckplatte hinter dem Magen mit dem Dorn des Staplers zu verbinden sowie der Gefahr der Verletzung dort möglicherweise unmittelbar liegender Strukturen wie Pankreas oder große Gefäße wird diese Operation üblicherweise offen-chirurgisch durchgeführt und in bislang nur wenigen Zentren weltweit laparoskopisch vorgenommen.

Bei bisher aus der DE-OS 33 00 768 und der DE-PS 33 01 713 bekannten chirurgischen Klammernahtinstrumenten zum Verbinden zweier Organwände ist eine Konnektierung von Dorn und Gegendruckplatte im wesentlichen von der Erfahrung und dem Tastsinn des Chirurgen abhängig und zuverlässig nur offenchirurgisch möglich.

Mit der Erfindung soll dem laparoskopisch versierten Chirurgen eine Vorrichtung zur funktionsgerechten Halterung einer Gegendruckplatte für ein schaftförmiges chirurgisches Klammernahtinstrument nach Art eines Zielgerätes verfügbar gemacht werden, das eine einfache und sichere Konnektierung des Klammernahtinstruments bzw. Circularstaplers und der Gegendruckplatte ermöglicht.

Erfindungsgemäß wird diese Aufgabe durch die in den Ansprüchen 1 bzw. 13 angegebenen Merkmale gelöst. Bevorzugte Merkmale, die die Erfindung vorteilhaft weiterbilden, sind in den jeweils nachgeordneten Patentansprüchen enthalten.

Aufgrund der erfindungsgemäßen Ausgestaltung der Vorrichtung wird dem laparoskopisch versierten Chirurgen erstmals ein sicheres Hilfsinstrument gegeben, das eine sichere und zuverlässige Konnektierung von Stapler und Gegendruckplatte sozusagen blind hinter dem Magen ermöglicht. Gleichzeitig bietet die erfindungsgemäße Konzeption die Voraussetzung für eine Gestaltung und Bauweise der Vorrichtung, die eine Verletzung von nicht zu operierenden sonstigen Organen und Gefäßen verhindert.

Die Vorrichtung läßt sich leicht an Schaftabschnitten von Circularstaplern bzw. Klammernahtinstrumenten anbringen und besteht vorteilhaft nur aus einem einteiligen Halteorgan, das aufgrund seiner Gestaltung einerseits ein Führen hinter die Magenwand erleichtert und andererseits durch seine verschiebbare und lösbare Anbringung am Schaftabschnitt sowie der lösbaren Aufnahme der Gegendruckplatte eine sichere und zuverlässige Ausrichtung und Halterung der Gegendruckplatte zur vorgesehenen Konnektierung vorsieht.

Gemäß einer bevorzugten Ausgestaltung der Erfindung ist das Halteorgan spangenförmig ausgebildet, wobei der Halterungsabschnitt für die Gegendruckplatte an einem aufgeweiteten Ende vorgesehen ist. Die spangenförmige Gestaltung verbindet die Vorzüge einer verletzungsarmen Ausbildung aus Rundprofilen mit denen einer einfachen Herstellbarkeit.

Vorzugsweise ist der Halterungsabschnitt für das Halteorgan mit zwei Ringhälften gebildet, in denen wenigstens eine Profileinformung zur wenigstens abschnittsweisen formschlüssigen Aufnahme der Außenkonturen der Gegendruckplatte vorgesehen ist. Diese Maßnahmen dienen der zuverlässigen Lagesicherung der Gegendruckplatte für die vorgesehene Konnektierung mit dem aus dem Stapler ausfahrbaren Dorn. Vorzugsweise sind dabei die Ringhälften gegen die Wirkung einer Vorspanneinrichtung aufspreizbar, die bevorzugt auch die Gegendruckplatte in den Halterungsabschnitt beaufschlagt, um zusätzlich zur formschlüssigen Aufnahme ein sicheres Halten zu gewährleisten.

Gemäß einer bevorzugten Ausgestaltung der Erfindung weist das Halteorgan einen schlittenförmigen Befestigungsteil auf, der an einem komplementären Profilabschnitt einer an einem distalen Schaftabschnitt des Klammernahtinstruments befestigbaren Halte- und Führungseinrichtung anbringbar, zwischen wenigstens zwei Raststellungen verschiebbar und von der Halte- und Führungseinrichtung lösbar ist. Aufgrund dieser Ausgestaltung wird vorteilhaft ermöglicht, daß für das Führen der Vorrichtung hinter den Magen das Halteorgan zunächst in einer ersten Raststellung angeordnet ist, in der es weiter von dem distalen Ende des Klammernahtinstrumentes beabstandet ist. Nach Erreichen der vorgesehenen Operationsstellung kann das Halteorgan dann in die zweite Raststellung näher zum distalen Ende des Klammernahtinstruments verschoben werden, um in den Eingriffsbereich des anzukoppelnden Dorns des Staplers zu gelangen. Nach erfolgter Konnektierung, Zusammenführung der Wände, Klammerung und Lochstanzung kann dann die Gegendruckplatte von der Halte- und Führungseinrichtung in einfacher Weise durch Zurückschieben des Halteorgans gelöst und anschließend das Halteorgan zur Entfernung aus dem Operationsbereich von der Halte- und Führungseinrichtung entfernt werden.

Das schlittenförmige Befestigungsteil des Halteorgans ist vorteilhaft mit zwei parallelen Spannenschenkeln des Halteorgans und horizontalen Querabschnitten gebildet. Vorzugsweise weist das schlittenförmige Befestigungsteil wenigstens zwei lösbare Rastorgane zur Sicherung von an der Halte- und Führungseinrichtung durch Rastaufnahmen gebildeten Raststellungen auf. Die Rastorgane können dabei vorteilhaft selbstrastende Organe, wie federbelastete Kugeln oder dgl., sein, die in komplementäre Ausnehmungen an der Halte- und Führungseinrichtung raststellungsabhängig eingreifen.

Zur Befestigung der Halte- und Führungseinrichtung ist vorteilhaft eine einfach anbringbare zweiteilige Befestigungshülse mit einem Schaftbefestigungsteil und einem Profilabschnitteil für das Befestigungsteil vorgesehen, wobei die beiden Befestigungshülsenteile wechselseitig ineinandergreifende Abschnitte aufweisen.

Für das Verschieben und das Lösen des Befestigungsteils ist nach einer bevorzugten Weiterbildung der Erfindung wenigstens ein bügelförmiges Handhabungsteil vorgesehen.

Zur Erleichterung der Entfernung des Halteorgans ist nach einer weiteren Ausgestaltung der Erfindung vorgesehen, das das Halteorgan an seinem dem Halterungsabschnitt entgegengesetzten Ende eine Einrichtung zur Befestigung eines Zugorgans aufweist.

Nach einer weiteren Ausgestaltung der Erfindung ist gemäß Anspruch 13 ein chirurgisches Klammergerät zur Herstellung zirkulärer Nähte vorgesehen, bestehend aus einem an einem distalen Schaftabschnitt 56 verschieb- und lösbar anbringbaren einteiligen Halteorgan 50 mit einem seitlich abgekröpften Abschnitt 54, der in einen Halterungsabschnitt 55 für die lösbare Aufnahme der Gegendruckplatte 51 mündet, wobei der Positionierung der Gegendruckplatte eine lösbar mit dem Geräteschaft verbindbare Zwangsführung zugeordnet ist. Insbesondere die Kopplung von Gegendruckplatte und Klammernahtinstrument mittels einer Zwangsführung gewährleistet hierbei, daß die Gegendruckplatte der Zugeinrichtung bzw. dem Klammermagazin lagerichtig zugeführt wird und eine einwandfreie Adaption der Gegendruckplatte mit der Zugeinrichtung gegeben ist. Die lösbare Anbindung der Führungseinrichtung an den Geräteschaft bewirkt, daß eine getrennte Einführung von Zwangsführung und Geräteschaft des Klammernahtinstrumentes durch ein Trokar ermöglicht ist. Zwangsführung und Geräteschaft werden dann innerhalb des Bauchraumes des Patienten miteinander verbunden. Die Trennung von Geräteschaft und Zwangsführung ist insofern unerläßlich, da der Durchmesser des Trokars meist nur geringfügig größer ist als der Durchmesser des Klammermagazins und eine Einführung des Geräteschaftes mit angekoppelter Führungseinrichtung daher in den meisten Anwendungsfällen unmöglich ist.

In einer zu bevorzugenden Ausgestaltungsform umfaßt die erfindungsgemäße Zwangsführung eine, vermittels einer Kupplungseinrichtung mit dem Geräteschaft verbindbare Führung, ein in dieser axial verschieblich aufgenommenes Führungsrohr sowie eine an dessen einem Ende angeordnete Halterung für die Aufnahme der Gegendruckplatte. Die zur Verbindung der Zwangsführung mit dem Geräteschaft dienende Kupplungseinrichtung besteht hierbei aus einem den Geräteschaft umgreifenden Befestigungsteil, welches in einer bevorzugten Ausführungsform als Klemmhülse ausgeführt ist, sowie einer Kupplungsplatte für eine Anbindung der Führung an die Kupplungseinrichtung. Für eine einfachere Einführung des Geräteschaftes in den Bauchraum des Patienten durch das Trokar ist daher auch vorgesehen, das Befestigungsteil und die Kupplungsplatte der Kupplungseinrichtung mittels zweier parallelogrammartig angeordneter Hebelpaare schwenkbar miteinander zu verbinden derart, daß die Kupplungsplatte an den Geräteschaft herangeschwenkt werden kann und sich der Umfang des Klammernahtinstrumentes im Bereich der Kupplungseinrichtung minimiert, wodurch eine Einführung des Klammernahtinstrumentes in den Bauchraum erleichtert wird. Hierzu ist vorteilhafterweise die dem Geräteschaft zugewandte Oberflächenkontur der Kupplungsplatte derart auf die Oberflächenkontur des Geräteschaftes abgestimmt, daß die Kupplungsplatte in der abgelegten Einführlage formschlüssig an dem Geräteschaft anliegt.

Die Hebelpaare der Kupplungseinrichtung können hierbei vermittels eines unmittelbar an der Kupplungsplatte angreifenden, mit einer Handhabe versehenen und am Geräteschaft bzw. Griff des Klammernahtinstrumentes abgestützten Verstellgestänges oder Seinzugeinrichtung verschwenkt werden. In Verbindung mit der Verwendung einer Seilzugeinrichtung ist dann weiterhin auch vorgeschlagen, für die Rückführung der Kupplungsplatte aus der an den Geräteschaft herangeschwenkten Einführstellung in die aufgerichtete Arbeitsstellung wenigstens eine Rückholfeder vorzusehen. Die Rückholfeder greift hierbei entweder an der Kupplungsplatte oder an wenigstens einem Hebelpaar an und ist an einem Befestigungsteil abgestützt.

In einer weiteren bevorzugten Ausgestaltungsform ist vorgesehen, daß die Rückführung der Kupplungseinrichtung in der Arbeitslage vermittels zweier parallel zueinander angeordneter, einerseits an der Anschlußplatte angreifender und andererseits mit dem Befestigungsteil verbundener Rückholfedern erfolgt.

In einer weiteren vorteilhaften Ausgestaltungsform ist die eine Hälfte der Kupplungseinrichtung mit dem Geräteschaft und die Halterung für die Gegendruckplatte mit dem Führungsrohr jeweils derart über Hebelpaare verbunden, daß eine Verschwenkung der Hebelpaare zu einer zwangsweisen zum Geräteschaft koaxialen Ausrichtung der Gegendruckplatte führt.

In Verbindung hiermit kann dann weiterhin auch vorgesehen sein, daß das der Kupplungseinrichtung zugeordnete Hebelpaar als zwei Parallelogrammhebel in Achsrichtung des Geräteschaftes übereinanderliegend und zwischen Geräteschaft diesem zugeordneter Kupplungshälfte der Kupplungseinrichtung angeordnet ist, während das der Halterung für die Gegendruckplatte zugeordnete Hebelpaar am Ende des Führungsrohres angeordnet ist, wobei die führungsrohrseitigen Anlenkachsen in Längsrichtung des Führungsrohres übereinanderliegend angeordnet sind.

Damit eine Einführung der Zwangsführung in den Trokar erleichtert wird, ist weiterhin vorgesehen, die Gegendruckplatte zusammen mit ihrer Halterung vermittels des an der Halterung angreifenden Hebelpaares aus einer parallel zu der Achse des Führungsrohres liegenden Arbeitsstellung in eine zu dem Führungsrohr koaxiale Einführlage zu verschwenken, wobei ein konzentrisch auf der Gegendruckplatte angeordneter Kupplungszapfen zwischen einem Hebelpaar aufgenommen wird.

Damit die Betätigung des der Halterung für die Gegendruckplatte zugeordneten Hebelpaares bei in den Trokar eingeführter Zwangsführung möglich ist, ist in einer bevorzugten Verwirklichungsform weiterhin vorgesehen, innerhalb des hohlen Führungsrohres ein Verstellgestänge anzuordnen, welches einerseits an einem der Halterung der Gegendruckplatte zugeordneten Parallelogrammhebel angreift und andererseits mit einer Handhabe zur manuellen Bedienung verbunden ist.

Zur Vermeidung einer unbeabsichtigten Auslenkung der Halteeinrichtung für die Gegendruckplatte aus der Arbeitslage ist zusätzlich vorgesehen, das Verstellgestänge mittels eines an dem Führungsrohr befestigten und einem am Verstellgestänge angreifenden Federmittels in einer der Arbeitsstellung der Halteeinrichtung der Gegendruckplatte entsprechenden Stellung vorzuspannen, so daß eine Auslenkung aus der Arbeitsstellung nur entgegen dieser Federkraft erfolgen kann.

Damit eine einwandfreie Funktion der Zwangsführung gewährleistet ist, ist eine lagegenaue Verbindung von Führung und Kupplungsplatte unerläßlich. Damit eine exakte Justierung der zu verbindenden Teile erfolgen kann, ist vorgesehen, daß an der Führung ein Zentrierstift angeordnet wird, welcher mit einer entsprechend geformten Zentrierbohrung in der Anschlußplatte zusammenwirkt.

Eine Fixierung der Führung an der Kupplungsplatte erfolgt dann vermittels an der Führung angeordneter Federklammern bzw. federnder Rastzungen, die in entsprechende Rastausnehmungen der Kupplungsplatte eingreifen.

Damit eine sichere Befestigung des Kupplungsteiles der Führung gewährleistet ist, ist an einer Stirnseite der Führung ein abgewinkeltes, die Kontur der Führung überragendes Abstützteil angeordnet, welches in eine entsprechend geformte Ausnehmung der Kupplungsplatte eingreift und die aus der Rastzungen- oder Rastfederbefestigung resultierenden Kräfte abstützt.

Als Alternative zu dem vorhergehenden Befestigungsvorschlag für die Führung ist vorgeschlagen, an zwei sich gegenüberliegenden Oberflächen der Führungshülse jeweils ein abgewinkeltes, über die Außenkontur der Führung hinausragendes Führungsblech anzuordnen, welches mit einer komplementär gestalteten Führungsfläche an der Anschlußplatte zusammenwirkt, wobei die Federzungen oder Federklammern derartig an der Führung angeordnet sind, daß die Wirkrichtung der Führungsbleche und der Haltefedern um 90° zueinander versetzt sind. In Verbindung mit dieser räumlichen Anordnung von Halteklammern und Führungsblechen hat es sich weiterhin auch als vorteilhaft erwiesen, eine Federklammer mit U-förmiger Querschnittform und etwa V-förmigen Abwinkelungen den etwa parallel zueinander verlaufenden Federenden zu verwenden.

Damit eine sichere Axial-Arretierung des Führungsrohres innerhalb der Führung ermöglicht wird, ist in einer weiteren vorteilhaften Ausgestaltungsform vorgeschlagen, an der Führung ein in radialer Richtung wirkendes federbelastetes Rastmittel, welches mit entsprechenden Rastvertiefungen oder Rastmarken am Schaft der Halteeinrichtung zusammenwirkt, vorzusehen. In einer bevorzugten Ausführungsform besteht dann das Rastmittel aus einer innerhalb einer radialen Bohrung im Führungsteil angeordneten druckfederbeaufschlagten Rastkugel.

Eine unbedingt notwendige Sicherung des Führungsrohres gegen eine Verdrehung wird erfindungsgemäß dadurch erreicht, daß die Führung das Führungsrohr formschlüssig umgreift und das Führungsrohr eine von einer Kreisform abweichende Querschnittsform aufweist. In einer besonders vorteilhaften Ausgestaltungsform weist das Führungsrohr dann eine ovale Querschnittsform auf.

Für eine sichere Festlegung ist die Halterung für die Gegendruckplatte im wesentlichen ringförmig gestaltet und weist zwei radial wirkende Klemmbacken auf, die vermittels zweier Achsabschnitte um eine zur Schwenkachse der Hebelpaare parallele Achse schwenkbar mit den Hebelpaaren verbunden sind.

Nachfolgend wird die Erfindung unter Bezugnahme auf die beigefügten Zeichnungen näher erläutert. Es zeigen:
- Fig. 1: eine Seitenansicht einer mit dem Geräteschaft eines Klammernahtinstrumentes verbundenen Zwangsführung;
- Fig. 2: eine Seitenansicht einer Zwangsführung des Geräteschaftes in Einführstellung;
- Fig. 3: eine Seitenansicht der Halteeinrichtung der Gegendruckplatte und das der Gegendruckplatte zugeordnete, am Ende des Führungsrohres angeordnete Hebelpaar;
- Fig. 4: eine vergrößerte Seitenansicht der Kupplungseinrichtung mit Betätigungseinrichtung für den Verschwenkmechanismus;
- Fig. 5: eine Seitenansicht der Kupplungseinrichtung mit Rückholfedern;
- Fig. 6: eine Draufsicht auf die Kupplungseinrichtung mit Rückholfedern;
- Fig. 7: eine Seitenansicht einer erfindungsgemäßen Führung;
- Fig. 8: eine Draufsicht auf die Führung gemäß Fig. 7;
- Fig. 9: eine Seitenansicht einer Kupplungsplatte für die Anbindung der Führung aus Fig. 7;
- Fig. 10: eine Seitenansicht einer weiteren Gestaltungsmöglichkeit einer Führung;
- Fig. 11: eine Draufsicht auf die Führung gemäß Fig. 10;
- Fig. 12: eine Seitenansicht einer Kupplungsplatte für die Anbindung der Führung gemäß Fig. 10;
- Fig. 13: eine Draufsicht auf eine Halteeinrichtung für eine Gegendruckplatte;
- Fig. 14: ein zweites Ausführungsbeispiel einer erfindungsgemäßen Vorrichtung in schematischer Darstellung und angebracht an einem distalen Ende einer Circularstaplers bzw. Klammernahtinstruments;
- Fig. 15: eine schematisierte Darstellung des Halterungsabschnitts des Halteorgans ohne Gegendruckplatte;
- Fig. 16: eine Darstellung des Halterungsabschnitts gemäß Fig. 15 mit aufgenommener Gegendruckplatte;
- Fig. 17: eine Explosionsansicht der Halte- und Führungseinrichtung;
- Fig. 18: eine vergrößerte schematisierte perspektivische Teilansicht des Befestigungsteils des Halteorgans; und
- Fig. 19: eine schematisierte Seitenansicht des Halteorgans mit aufgenommener Gegendruckplatte.

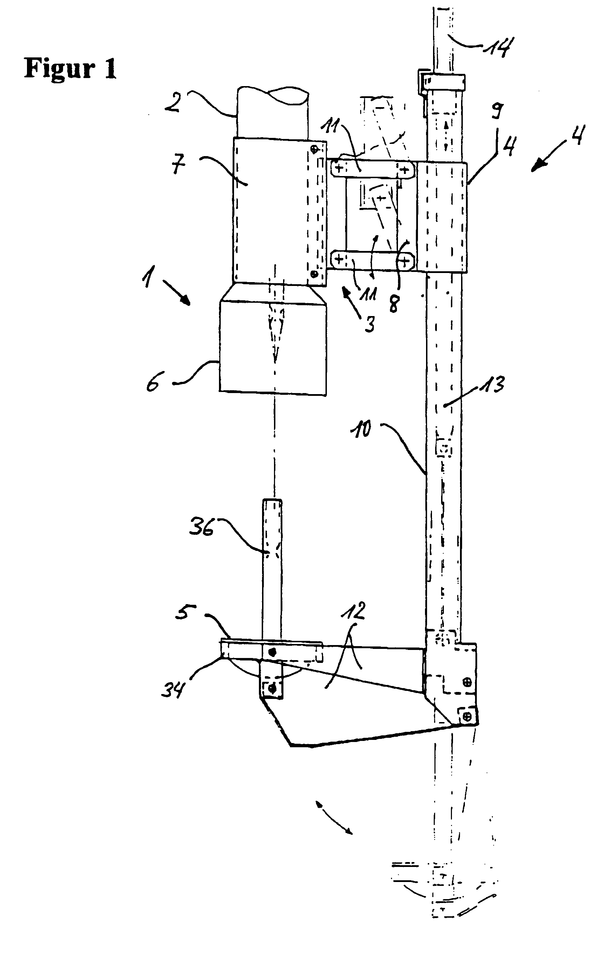

Die Fig. 1 zeigt eine am Geräteschaft 2 des Klammernahtinstrumentes 1 mittels einer Kupplungseinrichtung 3 befestigte Zwangsführung 4 für die Gegendruckplatte 5 mit Kupplungszapfen 36 des Klammernahtinstrumentes 1. Die Zwangsführung 4 ist unmittelbar oberhalb des Klammermagazins 6 mittels der Kupplungseinrichtung 3 an den Geräteschaft 2 angebunden. Die Kupplungseinrichtung 3 selbst besteht aus einem Befestigungsteil 7 zur Festlegung der Kupplungseinrichtung 3 am Geräteschaft 2 und einer Kupplungsplatte 8 für die Verbindung der Kupplungseinrichtung 3 mit der Führung 9 des Führungsrohres 10. Befestigungsteil 7 und Kupplungsplatte 8 sind über zwei Hebelpaare, die als Parallelogrammhebel 11 ausgebildet sind, schwenkbar miteinander verbunden.

In der mit der Kupplungseinrichtung 3 verbindbaren Führung 9 ist das Führungsrohr 10 axialverschieblich gelagert. Das Führungsrohr 10 trägt an seinem unteren Ende ein Hebelpaar 12, welches mit der Halterung 34 der Gegendruckplatte 5 verbunden ist. Dieses Hebelpaar 12 kann mittels eines innerhalb des Führungsrohres 10 angeordneten Verstellgestänges 13, welches mit einer Handhabe 14 versehen ist, betätigt werden, derart, daß die Halterung 43 für die Gegendruckplatte 5 zusammen mit der Gegendruckplatte 5 aus einer ihrer Arbeitsstellung entsprechenden Lage in eine zum Führungsrohr 10 koaxiale Einführlage verschwenkt werden kann, wie es in der Fig. 1 mittels gestrichelter Linien angedeutet ist.

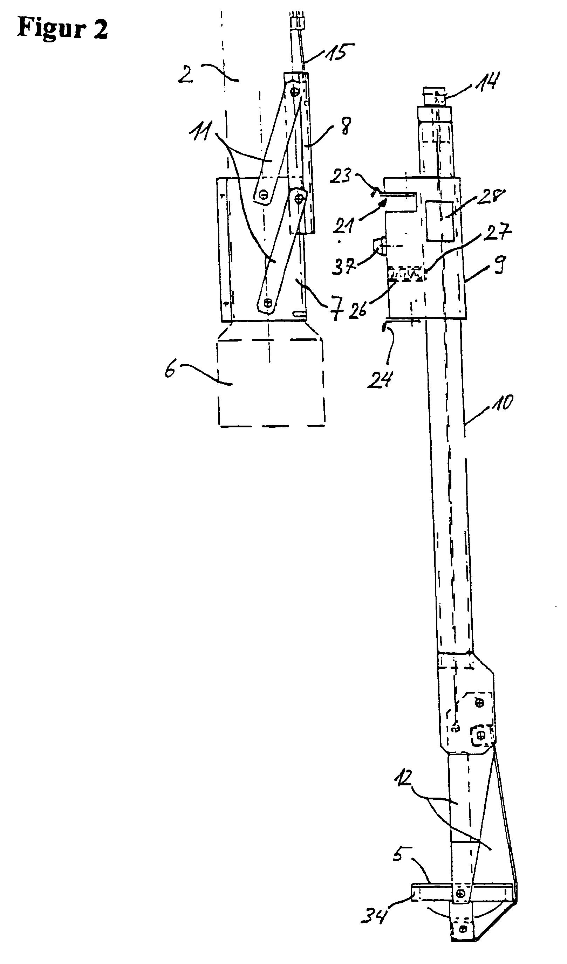

Für den Einführvorgang in den Bauchraum des Patienten wird, wie in Fig. 2 gezeigt, die Führung 9 von der Kupplungsplatte 8 getrennt und die Kupplungsplatte 8 mittels eines unmittelbar an der Kupplungsplatte 8 angreifenden Betätigungsgestänges 15 unter Mitwirkung der beiden Parallelogrammhebelpaare 11 an den Geräteschaft 2 herangeschwenkt. Die die Gegendruckplatte 5 tragende Halterung 34 wird vermittels des mit dem Führungsrohr 10 verbundenen Hebelpaares 12, welches über das im Führungsrohr 10 angeordnete Verstellgestänge 13 betätigt wird, aus der zum Geräteschaft 2 bzw. Klammermagazin 6 koaxialen Arbeitsstellung in eine zum Führungsrohr 10 koaxiale Einführstellung verschwenkt. Nach diesem Verschwenkvorgang können aufgrund des hierdurch verringerten Außenumfangs Führungsrohr 10, Führung 9 und Halterung 34 für die Gegendruckplatte 5 zusammen mit der Gegendruckplatte 5 fertigmontiert durch den Trokar in den Bauchraum des Patienten eingeführt werden. Anschließend wird der Geräteschaft 2 zusammen mit dem daran angeordneten Klammermagazin 6 und der auch am Geräteschaft 2 befestigten Kupplungseinrichtung 3 durch das Trokar in die Bauchhöhle eingeführt. Die Kupplungsplatte 8 ist während des Einführvorganges vermittels des Betätigungsgestänges 15 dicht an den Geräteschaft 2 herangeschwenkt, so daß sich in diesen Bereich des Geräteschaftes 2 eine verringerte Querschnittsfläche der Anordnung ergibt.

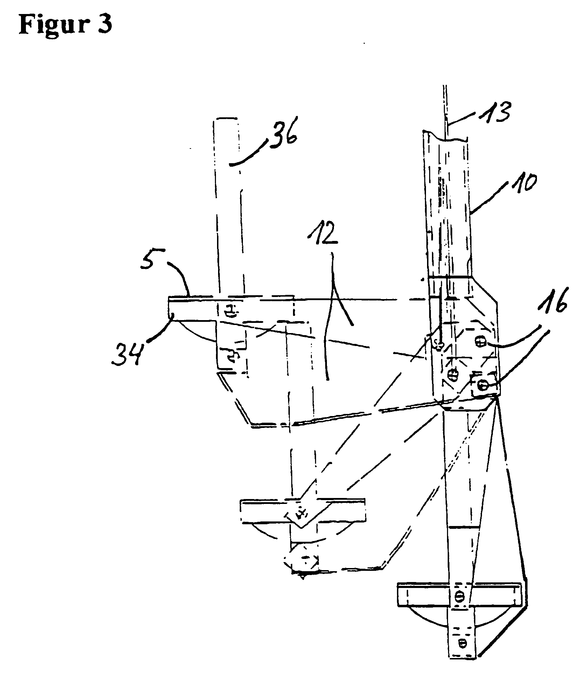

Innerhalb des Bauchraumes werden Kupplungsplatte 8 und Führung 9 für die Durchführung der Behandlung wieder miteinander verbunden. Anschließend wird die Gegendruckplatte 5 vermittels des innerhalb des Führungsrohres 10 angeordneten, am Hebelpaar 13 angreifenden Verstellgestänges 13 wieder aus der zum Führungsrohr 10 koaxialen Einführstellung in eine zum Geräteschaft 2 bzw. Klammermagazin 6 koaxiale Arbeitsstellung verschwenkt. Der Überführungsvorgang der Halteeinrichtung von einer zum Führungsrohr 10 koaxialen Einführstellung in eine zum Geräteschaft 2 koaxiale und zum Führungsrohr 10 parallele Lage ist auch in der Fig. 3 dargestellt. Aus dieser Fig. 3 ist weiterhin ersichtlich, daß die dem Führungsrohr 10 zugeordneten Gelenke 16 des Hebelpaares 13 in axialer Richtung des Führungsrohres 10 übereinanderliegend angeordnet sind.

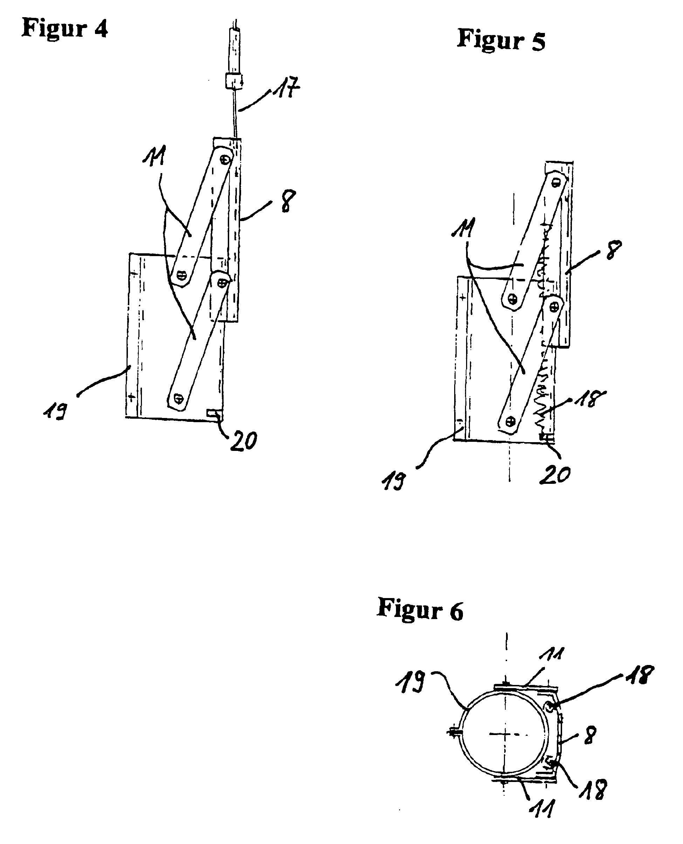

In Fig. 4 ist eine vergrößerte Darstellung der erfindungsgemäßen Kupplungseinrichtung gezeigt. Das Befestigungsteil 7 der Kupplungseinrichtung 3 ist hierbei als Klemmhülse 19, die mittels nicht dargestellter Klemmschrauben oder dgl. am Geräteschaft 2 festgelegt wird, ausgeführt. Die Verschwenkbewegung der Kupplungsplatten 8 erfolgt bei diesem Ausführungsbeispiel mittels eines an der Kupplungsplatte 8 angreifenden Bowdenzuges 17.

Wird die Auslenkung der Kupplungsplatte 8 aus der Arbeitsstellung mittels eines an der Kupplungsplatte 8 angreifenden Zugseiles bzw. Bowdenzug 17 erreicht, so müssen besondere Rückstellmechanismen vorgesehen werden, welche sicherstellen, daß die Kupplungsplatte 8 nach dem Einführen in den Bauchraum wieder in eine Arbeitslage zurückgeschwenkt wird. Wie insbesondere aus Fig. 5 und Fig. 6 ersichtlich, wird diese Rückstellung der Kupplungsplatte 8 vermittels zweier parallel zueinander angeordneter Zugfedern 18, welche an zwei beidseitig der Klemmhülse 19 angeordneten Parallelogrammhebeln 11 angreifen und an Nuten 20 der Klemmhülse 19 abgestützt sind, bewerkstelligt.

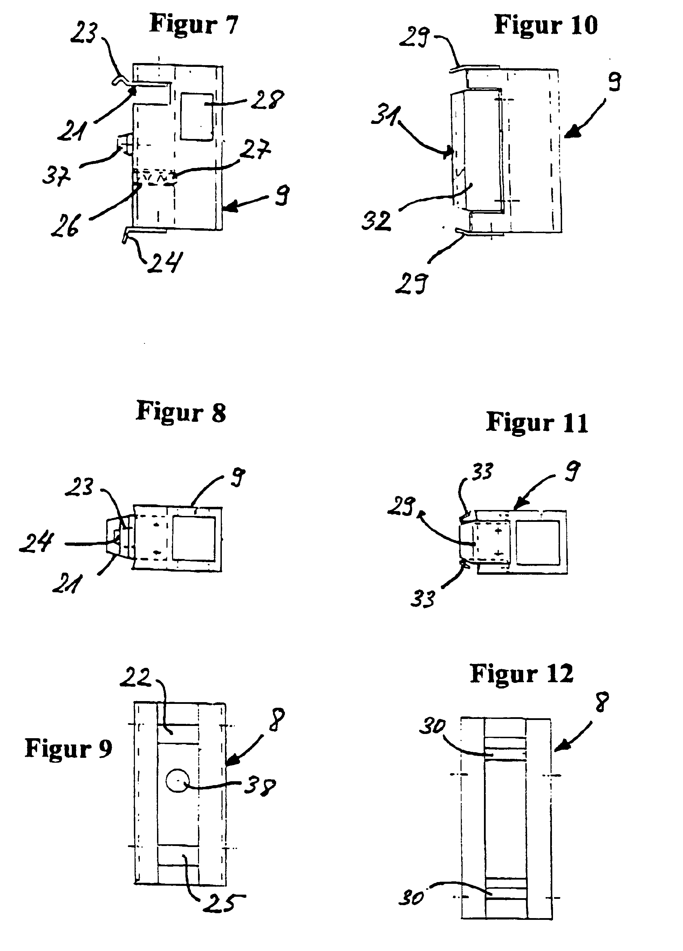

Die Führung 9 ist, wie aus Fig. 7 und Fig. 8 ersichtlich, vermittels einer an der Führung 9 angeordneten Rastfeder bzw. Rastzunge 21 an der Kupplungsplatte 8 gehalten, wobei die Rastfeder bzw. Rastzunge 21 in eine entsprechende Rastausnehmung 22 der Kupplungsplatte 8, wie in Fig. 9 gezeigt, eingreift und diese Rastausnehmung 22 der Kupplungsplatte 8 mittels eines an der Rastfeder oder Rastzunge 21 angeordneten Rastnockens 23 hintergreift. Die aus der Rastfeder- und Rastzungenbefestigung resultierenden Kräfte sind vermittels eines, an der dem Klammermagazin 6 zugewandten Stirnseite 35 der Führung 9 angeordneten, die Kontur der Führung 9 überragenden sowie abgewinkelten Abstützteiles 24, welches in eine entsprechend geformte Ausnehmung 25 der Kupplungsplatte 8 eingreift, aufgenommen. Eine exakte Justierung der Führung 9 an der Kupplungsplatte 8 ist dadurch erreicht, daß an der Führung 9 ein Zentrierstift 37 und an der Kupplungsplatte 8 eine mit dem Zentrierstift zusammenwirkende, komplementäre Zentrierbohrung 38 vorgesehen ist. Wie weiterhin aus Fig. 7 ersichtlich ist, erfolgt eine axiale Sicherung des Führungsrohres 10 innerhalb der Führung 9 vermittels einer innerhalb einer radialen Bohrung 26 angeordneten federbelasteten Rastkugel 27.

Der auch in Fig. 7 dargestellte rechteckige Durchbruch 28 an der Führung 9 dient der Erhöhung der Griffigkeit.

Eine alternative Ausgestaltungsform des Befestigungsmechanismus für die Befestigung einer Führung 9 ist in den Fig. 10 und Fig. 11 dargestellt. Hier sind an zwei sich gegenüberliegenden Oberflächen der Führung 9 zwei abgewinkelte, über die Außenkontur der Führung 9 hinausragende Führungsbleche 29 angeordnet. Diese Führungsbleche 29 wirken mit komplementär gestalteten Führungsflächen 30 an der Kupplungsplatte 8 zusammen und erleichtern den Montagevorgang beim Ankoppeln der Führung 9 an die Kupplungsplatte 8 innerhalb des Bauchraumes. Die Rastfeder 31 weist bei diesem Ausführungsbeispiel eine etwa U-förmige Querschnittsform auf, wobei an den Enden der beiden etwa parallel zueinander verlaufenden Federschenkel 32 eine V-förmige Abwinkelung 33 vorgesehen ist. Die Rastfeder 31 ist hierbei bezüglich der Führungsbleche 29 derart an der Führung 9 angeordnet, daß die Wirkrichtungen von Führungsblechen 29 und Federschenkeln 32 um ca. 90° Winkelgrade zueinander versetzt sind.

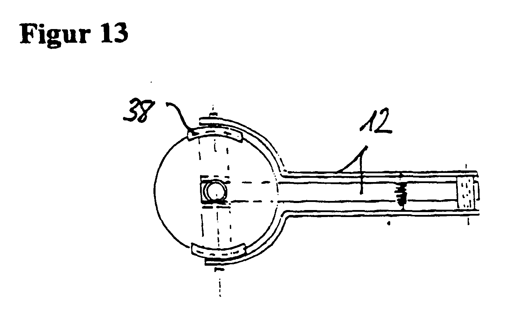

In Fig. 13 ist eine bevorzugte Klemmeinrichtung für die Fixierung der Gegendruckplatte an dem dieser Druckplatte zugeordneten Hebelpaar dargestellt. Diese Klemmeinrichtung besteht aus zwei radial wirkenden an dem oberen Hebel des Hebelpaares 12 angeordneten Klemmbacken 38.

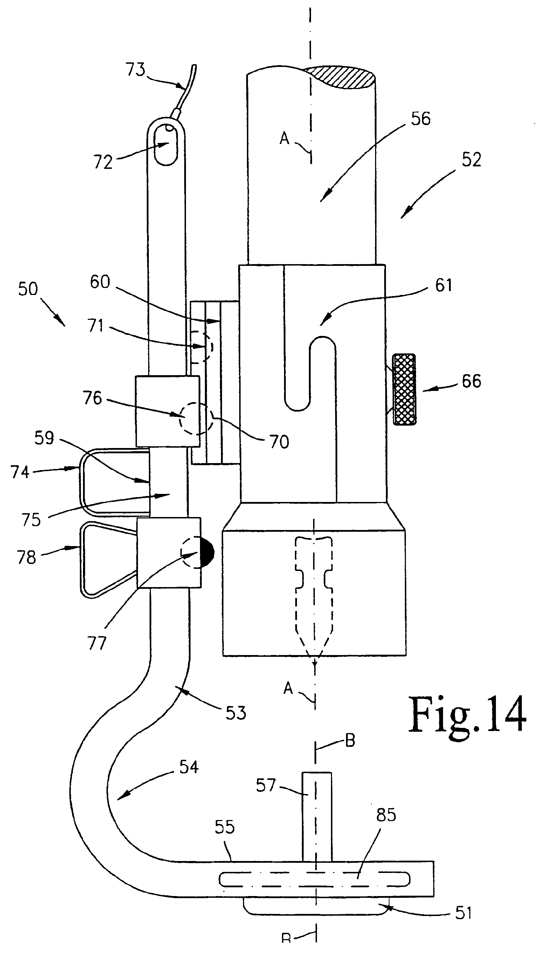

In den Fig. 14 bis 19 ist ein weiteres bevorzugtes Ausführungsbeispiel einer erfindungsgemäßen Vorrichtung 50 zur funktionsgerechten Halterung einer Gegendruckplatte 51 für ein schaftförmiges chirurgisches Klammernahtinstrument 52 dargestellt, wobei von dem Klammernahtinstrument 52 nur das untere Ende schematisiert dargestellt ist. Die Vorrichtung 50 ist nach Art einer Spange mit zwei Strangprofilabschnitten 53 einteilig gebildet und weist einen seitlich abgekröpften Abschnitt 54 auf, der in einen Halterungsabschnitt 55 für die lösbare Aufnahme der Gegendruckplatte 51 mündet. Der Halterungsabschnitt 55 erstreckt sich senkrecht zum Schaftabschnitt 56, wobei die Mitte bzw. die Mittelachse B-B der Gegendruckplatte 51, welche durch den senkrecht zur Gegendruckplatte angeformten Aufnahmeschaft 57 verläuft, mit der Schaftlängsachse A-A fluchtet.

Von dem Klammernahtinstrument 52 ist nur der untere Abschnitt schematisiert dargestellt, wobei ein aus dem hohlen Inneren des Endes ausfahrbarer Kupplungsdorn 58, der zur Konnektierung mit dem Schaft 57 der Gegendruckplatte 51 vorgesehen ist, überwiegend gestrichelt dargestellt ist.

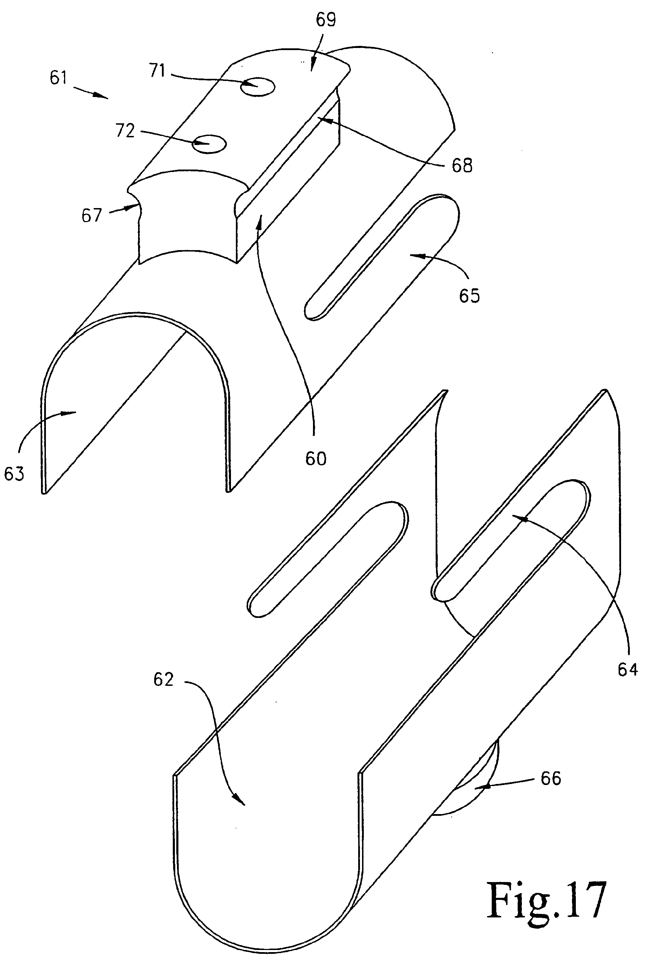

Das Halteorgan 50 weist einen schlittenförmigen Befestigungsteil 59 auf, der an einem komplementären Profilabschnitt 60 einer Halte- und Führungseinrichtung 61 anbringbar ist, welche an dem distalen Schaftabschnitt 56 des Klammernahtinstruments 52 befestigt ist und zwischen wenigstens zwei Raststellungen verschiebbar und von der Halte- und Führungseinrichtung 60 lösbar ist. Wie im Zusammenhang mit Fig. 17 erkennbar, besteht die Halte- und Führungseinrichtung 61 aus einer zweiteiligen Befestigungshülse mit einem Schaftbefestigungsteil 62 und einem Profilabschnitteil 63 für das Befestigungsteil 59. Die beiden Befestigungshülsenteile 62 und 63 besitzen wechselseitig ineinander greifende mäanderförmige Abschnitte 64 bzw. 65, die ein problemloses Aufklicken der Hülsenteile auf dem Schaft und eine sichere Verbindung beider Teile ermöglichen. Zusätzlich erfolgt noch eine Befestigung durch eine Rändelschraube 66.

Auf dem Profilabschnitteil 63 ist der Profilabschnitt 60 befestigt, der zwei seitliche Gleitführungsnuten 67 und 68 sowie eine Anlagefläche 69 mit Rasteinformungen 70 und 71 aufweist, die in einer Linie angeordnet sind, welche sich parallel zur Symmetrieachse A-A des Schaftes 56 des Klammernahtinstruments 52 erstreckt. Die Führungsfläche 69 ist bei dem dargestellten Ausführungsbeispiel quer gewölbt, und die Führungsaufnahmen 67 und 68 sind in ihrem Krümmungsradius auf den der Stangenprofile des spangenförmigen Halteorgans so abgestimmt, daß diese wenigstens teilflächig gehalten werden können.

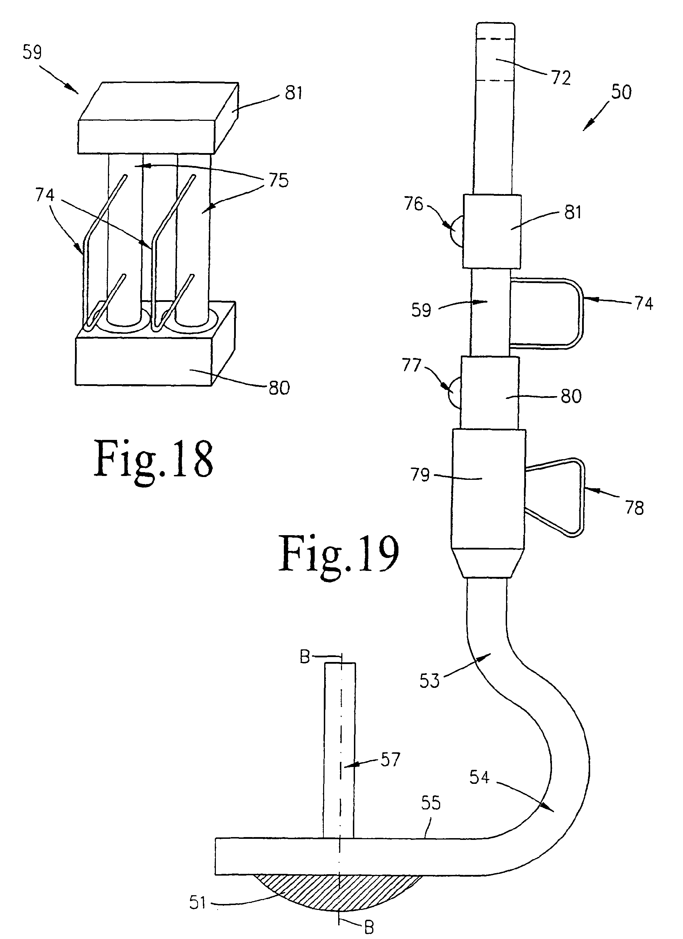

Das Halteorgan 50 erstreckt sich, wie in Fig. 10 gezeigt, über seinen Befestigungsabschnitt 59 bis zu einem oberen Ende, an dem eine lochförmige Aufnahme 72 zur Befestigung eines Zugorgans 73, beispielsweise einer Kordel, vorgesehen ist. Mit 74 sind, wie erläuternd in Fig. 18 dargestellt, zwei bügelförmige Handhabungsteile vorgesehen, die an zwei federgelagerten Profilabschnitten 75 des spangenförmigen Halteorgans 50 angebracht sind. Beim Zusammendrücken der beiden bügelförmigen Handhabungsteile 74 werden die Bolzenabschnitte 75 geringfügig auseinandergespreizt, um ein Aufstecken auf die Gleitprofile 67, 68 und 69 der Halte- und Führungseinrichtung 60 zu ermöglichen. Wie weiterhin der Fig. 19 zu entnehmen ist, weist der schlittenförmige Befestigungsteil 59 zwei druckfedergelagerte Rastkugeln 76 und 77 an der gegenüberliegenden Seite der bügelförmigen Handhabung 74 auf, wie schematisiert angedeutet. Die Rastkugeln 76 bzw. 77 sind für den Eingriff in die Rastausnehmung 70 bzw. 71 vorgesehen, um wenigstens zwei Raststellungen des Halteorgans 50 bezüglich des Klammernahtinstruments 52 zu sichern.

Mit 78 ist ein weiterer bügelförmiger Handhabungsteil angedeutet, der zur Vereinfachung in Fig. 18 weggelassen ist und in den Fig. 14 und 19 schematisch angedeutet ist. Dieser bügelförmige Handhabungsteil 78 ist in dem unteren Abschnitt des Befestigungsteils 59 befestigt und dient zur Bewegung des gesamten Halteorgans 50 über dessen schlittenförmiges Befestigungsteil 59 an der Halte- und Führungseinrichtung 60 und zum Lösen des Halteorgans 50.

Fig. 19 zeigt die Rückansicht des in Fig. 14 dargestellten Halteorgans 50, wobei das bügelförmige Handhabungsteil 78 an einem horizontalen Abschnitt 79 angebracht ist, der sich parallel zu den in Fig. 18 dargestellten horizontalen Führungsabschnitten 80 und 81 des schlittenförmigen Befestigungsteils 59 erstreckt.

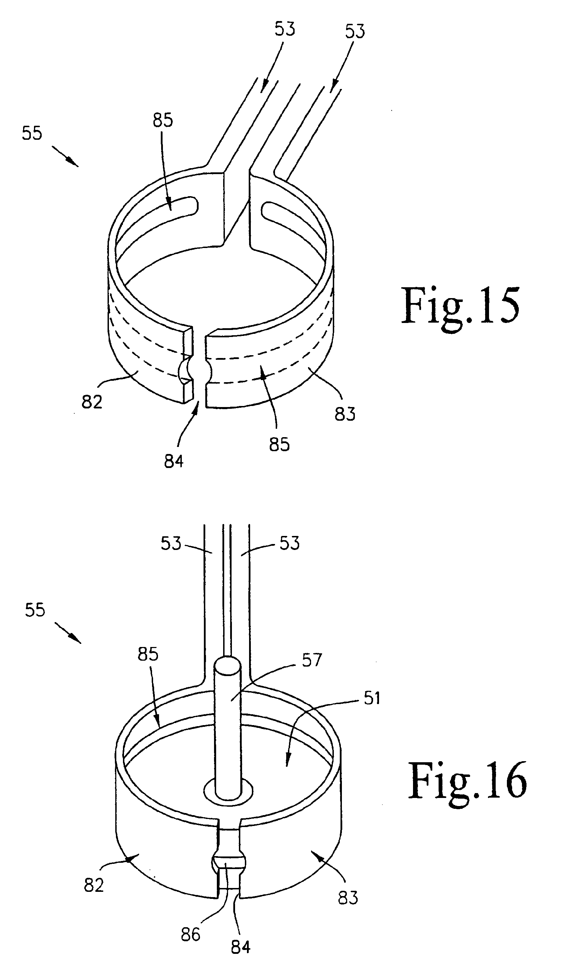

Wie im Zusammenhang mit Fig. 15 und 16 entnehmbar, ist der Halterungsabschnitt 55 mit zwei Ringsegmenten 82 und 83 gebildet, die an dem unteren Ende des spangenförmigen Halteorgans 50 angeformt sind. Zwischen den Ringabschnitten 82 und 83 befindet sich ein Spalt 84, der das Aufspreizen des Halterungsabschnitts 55 ermöglicht. Mit 85 ist eine nutförmige Profileinformung angedeutet, die zur formschlüssigen Aufnahme der Außenkontur 86 der Gegendruckplatte 51 dient, wie in Fig. 16 gezeigt. Die Gegendruckplatte 51 kann dabei von den Ringabschnitten 82 und 83 ohne zusätzliche die Ringabschnitte zusammendrückende Federkraft oder mit einer geringfügigen zusätzlich Federkraft aufgenommen sein.

Zur Handhabung der erfindungsgemäßen Vorrichtung 50 werden zunächst die beiden Hülsenteile 62 und 63 an den Schaft 56 des Circularstaplers bzw. Klammernahtinstruments 52 aufgeklickt und über den mäanderförmigen Verschluß 64, 65 miteinander verbunden. Anschließend erfolgt eine sichere Befestigung mittels der Rändelschraube 66.

Anschließend wird das Halteorgan 50 mit Hilfe seines schlittenförmigen Befestigungsteils 59 auf dem Profilabschnitt 60 des Profilabschnitteils 63 befestigt, indem die bügelförmigen Handhabungsteile 72 zueinander gedrückt und die zugeordneten bolzenförmigen Abschnitte 75 dadurch aufgespreizt und anschließend nach Aufsetzen auf den Profilabschnitt 60 in die Führungsnuten 67 und 68 zum Halten und Führen eingreifen. Dabei wird das Halteorgan 50 zunächst in der in Fig. 14 dargestellten Raststellung angebracht, in der der Halterungsabschnitt mit der darin angeordneten Gegendruckplatte 51 einen weitestmöglichen Abstand von dem distalen Ende des schaftförmigen Klammernahtinstruments 52 besitzt. Hierdurch wird ein leichtes Unterfahren der Magenhinterwand beim Einführen der Gegendruckplatte 51 mit ihrem angeformten Schaft 57 ermöglicht. Die Gegendruckplatte selbst wird ebenfalls noch außerhalb des Körpers in den Halterungsabschnitt 5 lagesicher mit Hilfe der formschlüssigen Halterung über die Nut 85 eingesetzt.

Anschließend wird dann über einen kleinen Hautschnitt im oberen Quadranten des Abdomens das konnektierte Halteorgan mit dem Circularstapler bzw. Klammernahtinstrument eingeführt, wobei vorher an der kleinen Kurvatur des Magens eine Lücke geschaffen wurde, in welche die Gegendruckplatte 51 eingeführt wird. Durch die besondere Form des Halteorgans mit seinem seitlich abgekröpften Abschnitt werden Vorder- und Hinterwand des Magens an der kleinen Kurvatur spangenförmig umfaßt, wobei sich die benötigten Abmessungen für den Magenpouch aus der Form des Halteorgans ergeben. Dies bedeutet, daß die seitliche Abkröpfung 54 des Halteorgans 50 unterschiedlich je nach Bedarf ausgestaltet sein kann, also sich auch weiter von der Längsachse A-A des Klammernahtinstruments entfernt ausbauchen kann.

Sobald die Gegenplatte "blind" hinter der Magenhinterwand von dem Chirurgen verankert worden ist, wird das Halteorgan 50 durch Ziehen an dem Zugorgan 73 in seine zweite Raststellung auf der Halte- und Führungseinrichtung verschoben. In dieser Position kann der Dorn 58 aus dem Kopf des Circularstaplers bzw. Klammernahtinstruments 52 ausgefahren werden und durchstößt die jeweiligen Magenwände, bis er in den Schaft 57 der Gegendruckplatte 51 zielgenau durch das Halteorgan 50 ausgerichtet gleitet. In dem Schaft 57 wird der Dorn 58 durch einen nicht dargestellten Federmechanismus zum sicheren Konnektieren festgehalten.

Durch Betätigung der bügelförmigen Handhaben 74 und 78 kann nun das Halteorgan von der Halte- und Führungseinrichtung 60 gelöst werden und kann dann einfach zunächst im OP-Gebiet abgelegt werden. Anschließend wird die Gegendruckplatte 51 endgültig an den Kopf des Circularstaplers herangezogen, wonach eine kreisförmige Klammernahtreihe gelegt und gleichzeitig ein Ring in die Magenvorderwand und die Magenhinterwand geschnitten wird. Anschließend wird der Circularstapler 52 mit den ausgeschnittenen Magenteilen aus dem Abdomen entfernt und schließlich über das Zugorgan 73 noch das Halteorgan 50 aus dem Abdomen herausgezogen.

## Patentansprüche

1. Vorrichtung zur funktionsgerechten Halterung einer einen Aufnahmeschaft (57) und eine durch diesen verlaufende Mittelachse (B-B) aufweisenden Gegendruckplatte (51) für ein schaftförmiges chirurgisches Klammernahtinstrument (52) zur Herstellung einer zirkulären Naht, bestehend aus einer Halte- und Führungseinrichtung (61), die an einem distalen Schaftabschnitt (56) des Klammernahtinstruments (52) anordenbar ist, und aus
einem einteiligen Halteorgan (50), das an der Halte- und Führungseinrichtung (61) verschiebbar und an dieser entfernbar befestigt ist, und das einen seitlich abgekröpften Abschnitt (54) aufweist,
wobei sich nach der Befestingung der Halte - und Fürhrungseinrichtung am Klammernaht instrument ein Halterungsabschnitt (55) senkrecht zum Schaftabschnitt (56) derart erstreckt, daß die Mittel einer Aufnahme (85) für die Gegendruckplatte bzw. Mittelachse (B-B) der Gegendruckplatte (51) mit der Schaftlängsachse des Klammernahtinstruments (A-A) fluchtet.

2. Vorrichtung nach Anspruch 1, **dadurch gekennzeichnet, daß** das Halteorgan (50) spangenförmig ausgebildet ist, wobei der Halterungsabschnitt (55) an einem aufgeweiteten Ende vorgesehen ist.

3. Vorrichtung nach Anspruch 1 oder 2, **dadurch gekennzeichnet, daß** der Halterungsabschnitt (55) des Halteorgans (50) zwei Ringhälften (82, 83) aufweist, in denen wenigstens eine Profileinformung (85) zur wenigstens abschnittsweisen formschlüssigen Aufnahme der Außenkontur (86) der Gegendruckplatte (51) vorgesehen ist.

4. Vorrichtung nach Anspruch 3, **dadurch gekennzeichnet, daß** die Ringhälften (82, 83) gegen die Wirkung einer Vorspannung aufspreizbar sind.

5. Vorrichtung nach Anspruch 3 oder 4 **dadurch gekennzeichnet, daß** die Ringhälften (82, 83) einen Aufnahmedurchmesser aufweisen, der geringer ist als die Außenkontur der Gegendruckplatte (51).

6. Vorrichtung nach einem der vorangehenden Ansprüche, **dadurch gekennzeichnet, daß** das Halteorgan (50) einen schlittenförmigen Befestigungsteil (59) aufweist, der an einem komplementären Profilabschnitt (60) der an einem distalen Schaftabschnitt (56) des Klammernahtinstruments (52) befestigbaren Halte- und Führungseinrichtung (61) anbringbar, zwischen wenigstens zwei Raststellungen verschiebbar und von der Halte- und Führungseinrichtung (61) lösbar ist.

7. Vorrichtung nach Anspruch 6, **dadurch gekennzeichnet, daß** der schlittenförmige Befestigungsteil (59) des Halteorgans (50) mit zwei parallelen Spangenschenkeln (53) des Halteorgans (50) und Querabschnitten (79, 80, 81) gebildet ist.

8. Vorrichtung nach Anspruch 6 oder 7, **dadurch gekennzeichnet, daß** der schlittenförmige Befestigungsteil (59) wenigstens zwei lösbare Rastorgane (76, 77) zur Sicherung von an der Halte- und Führungseinrichtung (61) durch Rastaufnahmen (70, 71) gebildeten Raststellungen aufweist.

9. Vorrichtung nach einem der Ansprüche 6 bis 8, **dadurch gekennzeichnet, daß** die Halte- und Führungseinrichtung (61) eine zweiteilige Befestigungshülse mit einem Schaftbefestigungsteil (62) und einem Profilabschnitteil (63) für den Befestigungsteil (59) aufweist.

10. Vorrichtung nach Anspruch 9, **dadurch gekennzeichnet, daß** die beiden Befestigungshülsenteile (62, 63) wechselseitig ineinandergreifende Abschnitte (64, 65) aufweisen.

11. Vorrichtung nach einem der Ansprüche 6 bis 10, **dadurch gekennzeichnet, daß** für das Verschieben und Lösen des Befestigungsteils (59) ein bügelförmiges Handhabungsteil (74) vorgesehen ist.

12. Vorrichtung nach einem der vorangehenden Ansprüche, **dadurch gekennzeichnet, daß** das Halteorgan (50) an seinem dem Halterungsabschnitt entgegengesetzten Ende eine Einrichtung (72) zur Befestigung eines Zugorgans (73) aufweist.

## Claims

1. An apparatus for the functionally appropriate mounting of a counter-pressure plate (51) for a shaft-type surgical clamp suturing instrument (52) for producing a circular suture, said counter-pressure plate having a receiving shaft (57) and a central axis (B-B) running through the latter, said apparatus consisting of a mounting and guide device (61) which can be disposed on a distal shaft portion (56) of the clamp suturing instrument (52), and
of a single-piece mounting element (50) which is attached in a movable and detachable manner to the mounting and guide device (61) and includes a laterally offset portion (54),
wherein, following attachment of the mounting and guide device to the clamp suturing instrument, a mounting portion (55) extends perpendicularly relative to the shaft portion (56) such that the centre of a receiver (85) for the counter-pressure plate, or the central axis (B-B) of the counter-pressure plate (51), is in alignment with the longitudinal axis (A-A) of the shaft of the clamp suturing instrument.

2. The apparatus according to claim 1, **characterized in that** the mounting element (50) is hook-shaped, the mounting portion (55) being provided on a widened end thereof.

3. The apparatus according to either of claims 1 or 2, **characterized in that** the mounting portion (55) of the mounting element (50) includes two half-rings (82, 83) in which are provided at least one profiled recess (85) for receiving at least portions of the outer circumference (86) of the of the counter-pressure plate (51) in a form-fitting manner.

4. The apparatus according to claim 3, **characterized in that** the half-rings (82, 83) can be spread apart against the action of a bias.

5. The apparatus according to either of claims 3 or 4, **characterized in that** the half-rings (82, 83) have a receiving diameter which is less than the outer circumference of the counter-pressure plate (51).

6. The apparatus according to any one of the preceding claims,
**characterized in that** the mounting element (50) includes a slide-type fastening part (59) which can be mounted on a complementary profiled portion (60) of the mounting and guide device (61) attachable to a distal shaft portion (56) of the clamp suturing instrument (52), can be moved between at least two engagement positions and can be released from the mounting and guide device (61).

7. The apparatus according to claim 6, **characterized in that** the slide-type fastening part (59) of the mounting element (50) is formed with two parallel hook-type limbs (53) of the mounting element (50) and of transverse portions (79, 80, 81).

8. The apparatus according to either of claims 6 or 7, **characterized in that** the slide-type fastening part (59) includes at least two releasable engagement elements (76, 77) for securing engagement positions realized on the mounting and guide device (61) by means of engagement receivers (70, 71).

9. The apparatus according to any one of claims 6 to 8, **characterized in that** the mounting and guide device (61) includes a two-part fastening sleeve comprising a shaft-fastening part (62) and a profile-portion part (63) for the fastening part (59).

10. The apparatus according to claim 9, **characterized in that** the two fastening-sleeve parts (62, 63) have mutually engaging portions (64, 65).

11. The apparatus according to any one of claims 6 to 10, **characterized in that** a bow-shaped handling part (74) is provided for moving and releasing the fastening part (59).

12. The apparatus according to any one of the preceding claims,
**characterized in that** the mounting element (50) includes, at its end opposite the mounting portion, means (72) for connection of a pull element (73).

## Revendications

1. Dispositif pour fixer de manière fonctionnelle une plaque de contre-pression (51) présentant une tige de logement (57) et un axe médian (B-B) passant par ladite tige, destinée à un instrument de suture chirurgical en forme de tige servant à poser des agrafes (52) en vue de réaliser une suture circulaire, comprenant un dispositif de retenue et de guidage (61) qui peut être disposé sur une partie tige distale (56) de l'instrument de suture servant à poser des agrafes (52) et
un organe de retenue (50) constitué d'une seule pièce qui peut être fixé de manière coulissante sur le dispositif de retenue et de guidage (61) et de manière démontable sur celui-ci et qui présente une partie coudée latéralement (54),
dans lequel, après la fixation du dispositif de retenue et de guidage sur l'instrument de suture servant à poser des agrafes, une partie de retenue (55) s'étend perpendiculairement à la partie tige (56) de telle manière que le centre d'un logement (85) pour la plaque de contre-pression, respectivement l'axe médian (B-B) de la plaque de contre-pression (51) est aligné avec l'axe longitudinal (A-A) de la tige de l'instrument de suture servant à poser des agrafes.

2. Dispositif selon la revendication 1, **caractérisé en ce que** l'organe de retenue (50) est en forme d'agrafe, la partie de retenue (55) étant prévue à une extrémité élargie.

3. Dispositif selon la revendication 1 ou 2, **caractérisé en ce que** la partie de retenue (55) de l'organe de retenue (50) présente deux moitiés d'anneau (82, 83) dans lesquelles au moins une forme profilée vers l'intérieur (85) est prévue pour loger au moins en partie de manière solidaire avec correspondance de forme le contour extérieur (86) de la plaque de contre-pression (51).

4. Dispositif selon la revendication 3, **caractérisé en ce que** les moitiés d'anneau (82, 83) peuvent s'écarter à l'encontre de l'action d'une précontrainte.

5. Dispositif selon la revendication 3 ou 4, **caractérisé en ce que** les moitiés d'anneau (82, 83) présentent un diamètre de logement qui est plus petit que le contour extérieur de la plaque de contre-pression (51).

6. Dispositif selon l'une des revendications précédentes, **caractérisé en ce que** l'organe de retenue (50) présente une pièce de fixation en forme de coulisseau (59) qui peut être placée sur une partie profilée complémentaire (60) du dispositif de retenue et de guidage (61) pouvant être fixé sur une partie tige distale (56) de l'instrument de suture servant à poser des agrafes (52), être coulissante entre au moins deux positions d'enclenchement et être séparable du dispositif de retenue et de guidage (61).

7. Dispositif selon la revendication 6, **caractérisé en ce que** la pièce de fixation en forme de coulisseau (59) de l'organe de retenue (50) est formée avec deux branches d'agrafe parallèles (53) de l'organe de retenue (50) et des parties transversales (79, 80, 81).

8. Dispositif selon la revendication 6 ou 7, **caractérisé en ce que** la pièce de fixation en forme de coulisseau (59) présente au moins deux organes d'enclenchement séparables (76, 77) afin de bloquer des positions d'enclenchement formées par des logements d'enclenchement (70, 71) sur le dispositif de retenue et de guidage (61).

9. Dispositif selon l'une des revendications 6 à 8, **caractérisé en ce que** le dispositif de retenue et de guidage (61) présente une douille de fixation en deux parties avec une partie de fixation de tige (62) et une partie de segment profilé (63) pour la pièce de fixation (59).

10. Dispositif selon la revendication 9, **caractérisé en ce que** les deux parties de douille de fixation (62, 63) présentent des segments (64, 65) venant en prise mutuellement les uns avec les autres.

11. Dispositif selon l'une des revendications 6 à 10, **caractérisé en ce qu'**il est prévu une pièce de manipulation (74) en forme d'étrier pour le coulissement et la séparation de la pièce de fixation (59).

12. Dispositif selon l'une des revendications précédentes, **caractérisé en ce que** l'organe de retenue (50) présente, à son extrémité opposée à la partie de retenue, un dispositif (72) destiné à la fixation d'un organe de traction (73).
